# NEW EUROPEAN PATENT SPECIFICATION

(11) **EP 0 601 610 B2**
(45) Date of publication and mention of the opposition decision: **11.08.2004**
(45) Mention of the grant of the patent: 06.05.1999
(21) Application number: 93120004.2
(22) Date of filing: 10.12.1993
(51) Int. Cl.: A61F 13/15

(54) **Absorbent article**
Absorbierender Artikel
Article absorbant

(30) Priority: 11.12.1992 JP 35240992
(43) Date of publication of application: 15.06.1994
(73) Proprietor: JAPAN ABSORBENT TECHNOLOGY INSTITUTE, Chuo-ku, Tokyo (JP)
(72) Inventor: Suzuki, Migaku, Kanagawa (JP); Fukui, Hiroaki, Kawaguchi-shi, Saitama (JP)
(74) Representative: Strehl Schübel-Hopf & Partner

(56) References cited:
- DE-A- 3 343 622
- US-A- 2 596 127
- US-A- 3 608 551
- US-A- 4 842 596

## Description

### FIELD OF THE INVENTION

The present invention generally relates to absorbent articles such as infant or adult diapers, and more particularly to an absorbent article incorporating an absorbent member that is connected to an elastic support being fixed to the front and the rear section of the outer cover of the absorbent article.

### BACKGROUND OF THE INVENTION

The disposable absorbent articles, such as infant or adult disposable diapers, are generally categorized into two types; a closed-type article such as a training pant, and an open-type article having a flat configuration which includes front, crotch and rear sections. Either of those absorbent articles typically comprises a liquid impermeable backsheet, a liquid permeable topsheet, and an absorbent core interposed therebetween, and are designed to effectively absorb and retain waste materials.

US-A-4 842 596 discloses a disposable absorbent garment having an absorbent structure superposable on the outer cover of said garment.

However, one drawback of such conventional integral absorbent articles is that they tend to be displaced away from a wearer's body by movement thereof. The displacement creates a space between the body and the absorbent article through which the waste materials leak. As a result, the desired absorbency of the absorbent core can not be fully utilized.

One attempt to overcome such drawback is illustrated in Japanese Kokai Patent No. Hei 3-202056. The Kokai patent proposes an absorbent article having a liquid impermeable stretchable topsheet with an aperture. A void space is provided between top and back sheets to improve conformability of the top sheet to the wearer's body and also to receive the waste materials therein which have passed through the aperture in the top sheet. This arrangement does not provide a better conformability of the absorbent core to the wearer's body since the topsheet is urged away from the absorbent core.

US-A-3 608 551 discloses short panties having a liquid impermeable barrier and a narrow elastic tape which is fixed to the front section and the rear section of said panties. An absorbent member, e.g. a napkin, can be attached to the tape and can be removed after use.

EP-A-0 386 816 discloses a diaper having a liquid impervious backsheet and a liquid pervious topsheet which is at least partially peripherally joined to said backsheet. The topsheet is elastically extensible and can be elongated to conform the shape of the wearer. An absorbent core is disposed between the topsheet and the backsheet.

It is an object of the present invention to provide an absorbent article which is able to accommodate the body movement of a wearer for providing continuous conformity of an absorbent member to the wearer's body.

### SUMMARY OF THE INVENTION

The present invention provides a disposable absorbent article according to claim 1

In a particular embodiment of the present invention, the elongated element comprises a liquid permeable facing sheet, and a liquid impermeable elastic backing sheet intermittently secured to the facing sheet to form a plurality of channels therebetween. The absorbent material may be placed in selected ones of the channels.

One of the front and rear support members is elastically stretchable and contractable. Preferably, the front support member may be elastically stretchable and contractable.

In one embodiment of the present invention, both of the front and rear supports are elastically stretchable and contractable. In a particular embodiment, the elastically stretchable support member includes a plurality of parallel slits respectively extending longitudinally form a longitudinal end thereof to form a plurality of lips separated by the slits, and each of said lips is secured to the outer cover by a spot of securement.

Numerous other advantages and features of the present invention will become readily apparent from the following detailed description, the appended drawings, and the accompanying claims.

### BRIEF DESCRIPTION OF THE DRAWINGS

FIGURE 1 is a perspective view of an absorbent article comprising a disposable diaper embodying the principles of the present invention;
FIGURE 2 is longitudinal cross-sectional view of the absorbent article of FIGURE 1;
FIGURE 3 is an enlarged, fragmentary perspective view of an elastic support;
FIGURE 4 is diagrammatic, longitudinal cross-sectional view of an elongated member incorporating absorbent material and elastic supports;
FIGURE 5 is diagrammatic plan view of an elongated member comprising an absorbent member and elastic supports;
FIGURE 6 is diagrammatic plan view of an elongated member illustrating a central cutout in each of the elastic supports;
FIGURE 7 is a fragmentary plan view of the elastic support illustrating a plurality of parallel slits formed therein; and
FIGURE 8 is a explanatory view of the elastic support of FIGURE 7 illustrating that the slits are caused to open up in responsive to the stretching of the outer cover.

### DETAILED DESCRIPTION

While the present invention is susceptible of embodiments in various forms, there are shown in the drawings and will hereinafter be described various embodiments of the invention, with the understanding that the present disclosure is to be considered as an exemplification of the invention, and is not intended to limit the invention to the specific embodiments illustrated and described herein.

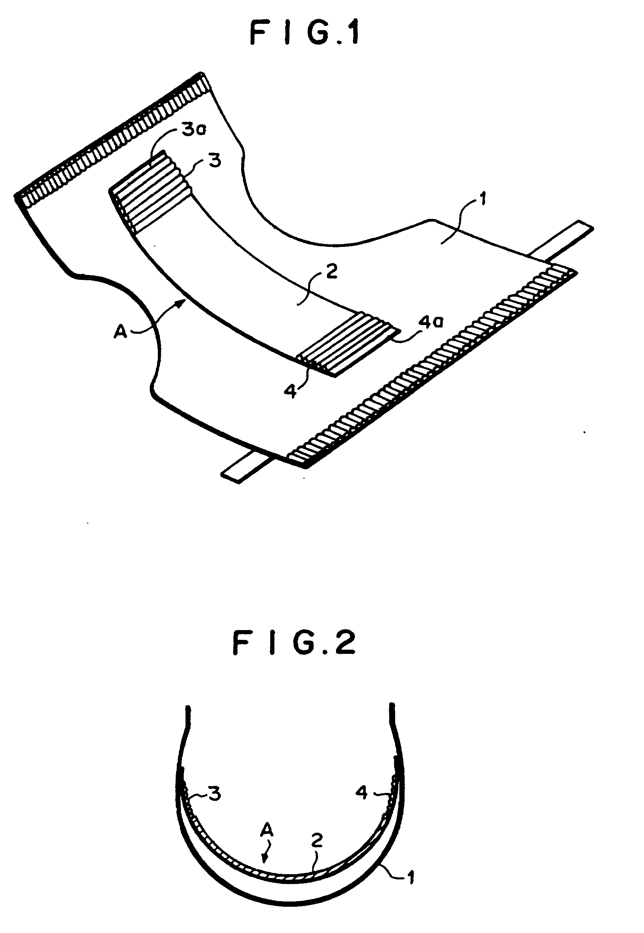

FIGURE 1 is a perspective view of a disposable absorbent article comprising a disposable diaper in accordance with the present invention. The absorbent article comprises a liquid impermeable outer cover 1, an absorbent member 2 supported on the outer cover 1, and one or more elastic elements for connecting at least one of front and rear ends, preferably the front end, more preferably the both of front and rear ends of the absorbent member 2 to the outer cover 1 in a suspended manner. In the illustrated embodiment, the elastic element comprises a first elastic support 3 for connecting the front end of the absorbent member 2 to a front section of the outer cover 1, and a second elastic support 4 for connecting the rear end of the absorbent member 2 to a rear section of the outer cover 1. Although the outer cover 1 is illustrated as being an open-type having a flat configuration, it may be a closed-type having a pant-like configuration.

The absorbent member 2 may comprise absorbent material which has been conventionally incorporated in disposable diapers or sanitary napkins. Alternatively, the absorbent member 2 may comprise a facing sheet, a backing sheet and absorbent material interposed between the facing and backing sheets. The absorbent member 2 may further have a side barrier structure so as to be self-functional as an absorbent product. The generally rectangular, first elastic support 3 is at its one end connected to the front end of the absorbent member 2 by suitable connecting means, such as heat-sealing. The other ends of the first elastic support 3 is connected to the outer cover 1 at a selected, rectangular region of securement 3a thereon. Likewise, the second elastic support 4 is at its one end connected to the rear end of the absorbent member 2, and is at its other end connected to the outer cover 1 at a selected, rectangular region of securement 4a on the outer cover 1.

The absorbent member 2 is of rectangular shape, having substantially the same width as the respective first and second elastic supports 3, 4 to together define an elongated strip-like element A. The elongated strip-like element A is suspended and extends longitudinally between front and rear sections of the outer cover 1. As illustrated in FIGURE 2, the strip-like element A has a length such that an intermediate length portion thereof is positioned in a spaced relationship with the outer cover 1. More specifically, the strip-like element A is supportedly positioned relative to the outer cover 1 in a hammock-like manner so that the absorbent member 2 does not contact the outer cover 1. Accordingly, when the absorbent article is actually applied to the wearer, the absorbent member 2 is urged toward a crotch area of the wearer's body for close contact therewith by elastically stretching and contracting actions of the respective first and second elastic supports 3,4.

The first and second elastic supports 3,4 respectively elastically stretch and contract responsive to any movement of the wearer's body during the application of the absorbent article thereto, so that an optimum position of the absorbent member 2 is maintained relative to the wearer's body. This ensures that the absorbent capacity of the absorbent member 2 can be fully utilized. In a specific absorbent article construction wherein the outer cover 1 comprises an elastic material, the absorbent article tends to readily deform as the wearer's body moves. Even in such a construction, the optimum position of the absorbent member 2 can be still maintained relative to the wearer's body in accordance with the present invention. This permits the outer cover 1 to comprise an elastic material for providing a better feeling during use.

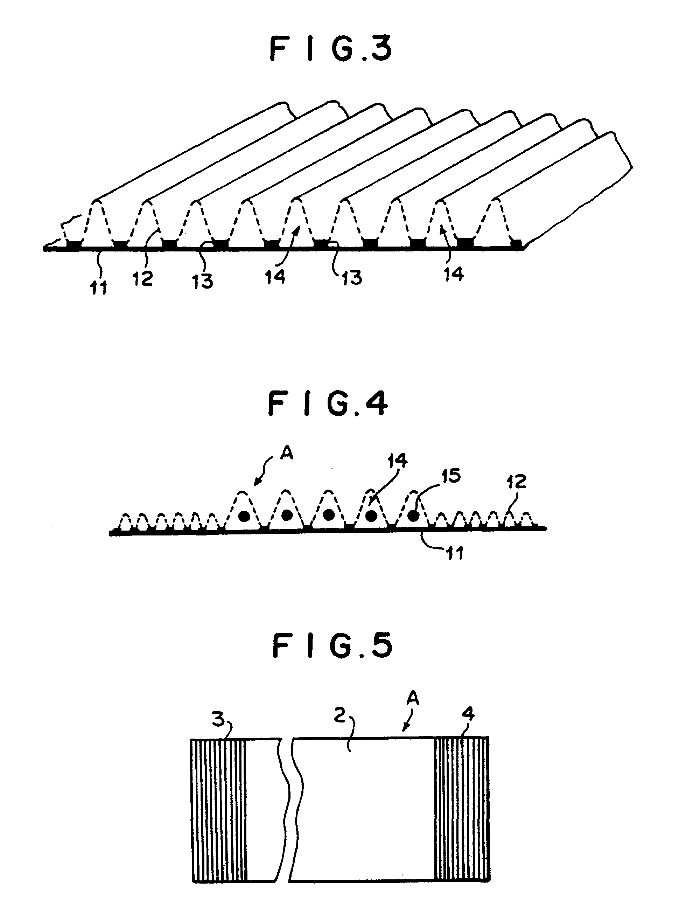

The elastic supports 3,4 of the present invention may comprise any suitable elastic material known in the art. Preferably, an elastic composite sheet as illustrated in FIGURE 3 may be employed. The elastic composite sheet comprises an elastic material sheet 11, and a non-woven fabric 12 which is secured to at least one surface of the elastic material sheet 11 continuously in a first direction and intermittently in a second direction transverse to the first direction. The securement of these two elements is made along parallel securement lines 13 spaced from each other. The width of the non-woven fabric 12 is formed to be greater than that of the elastic sheet material 11 between adjacent securement lines 13, so that a plurality of parallel channels 14 are formed between the elastic material sheet 11 and the non-woven fabric 12. The elastic composite sheet is capable of stretching to the extent that the elastic material sheet 11 is extended to the same width as the non-woven fabric 12. A corrugated configuration of the soft non-woven fabric 12 further provides a comfortable feeling by softness to skin.

Suitable materials for the elastic material sheet 11 include a thin-layered sheet such as of natural or synthetic rubber, polyurethane film, polyurethane melt-blown non-woven fabric, styrene-butadien block polymer film, or polyolefin elastomer film. When material cost, and adhesiveness to the non-woven fabric are taken into consideration, desirable materials include polyolefin elastomers such as EVA, LLDPE of ultra low density, ethylene propylene elastomer, ethylene-methyl-acrylate elastomer, or a blended combination of any of those polyolefin elastomers with synthetic rubber or styrene-ethylene-butadien styrene block polymer (SEBS), or co-extruded film of polyurethane elastomers and polyolefin elastomers.

FIGURE 4 illustrates one embodiment of the elongated, strip-like element A which integrally incorporates the absorbent member 2 and the first and second elastic supports 3,4. The elongated member A comprises the elastic composite sheet, as shown in FIGURE 3, with absorbent material 15 being placed in the channels 14 of the elastic composite sheet. In the illustrated particular embodiment, the channels 15 in a middle portion of the elastic composite sheet are configured to have a greater cross-sectional area than the remaining channels for enclosing the absorbent material 15 therein. The middle portion serves as the absorbent member 12, and end portions on opposite sides of the middle portions serve as the elastic supports 3,4. The absorbent material may preferably comprise superabsorbent material, such as superabsorbent polymer which is capable of absorbing a large volume of liquids.

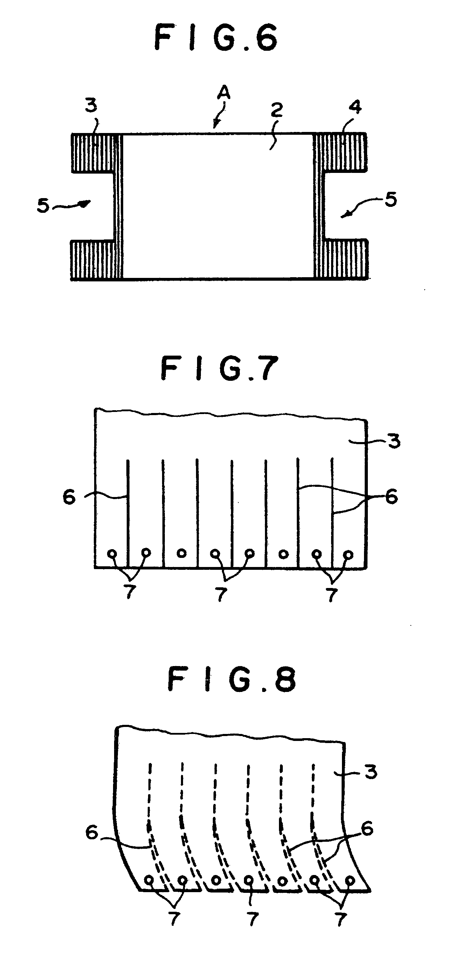

The illustrated elastic composite sheet for the elastic supports 3,4 has a relatively high stretchability in a direction transverse to the channel direction, and a relatively low stretchability in the channel direction which is limited to the stretching characteristics of the non-woven fabric 15. Accordingly, when the stretchability of the outer cover 1 is limited, the elastic composite sheet may have the same width as the absorbent member 2, and may be joined to the outer cover 1 over its full width as shown in FIGURE 5. However, when the outer cover 1 is made from a highly stretchable material, a central cutout 5 (FIGURE 6) may be preferably made in each of the first and second elastic supports 3,4 to form a reduced area for securement to the outer cover 1, so that the stretchability of the outer cover 1 is less inhibited. In other words, the central cutouts 5 of the respective elastic supports 3,4 serve to maintain the stretchability of the corresponding portions of the outer cover 1.

Another embodiment for joining at least one of the elastic supports 3,4 is illustrated in FIGURE 7. A plurality of parallel slits 6 are formed at selected intervals to respectively extend from one end of the elastic support 3 in a direction transverse to the channel direction for defining a plurality of lips separated by the parallel slits 6. Each of the lips is secured to the outer cover 1 by a spot of securement 7. As the outer cover 1 is stretched, each of slits 6 is accordingly opened up as illustrated in FIGURE 8, so that the outer cover 1 is able to stretch between the adjacent spots of securement 7. As such, the stretchability of the outer cover 1 is less
inhibited.

## Claims

1. A disposable absorbent article comprising a liquid impermeable outer cover (1) having a front section and a rear section and an absorbent member (2) disposed at the inner side of said outer cover (1), **characterized in that** an elastic support member (3, 4) is connected to one longitudinal end or two elastic support members are connected to both longitudinal ends of said absorbent member (2) to form an elongated element (A) having a front end, a rear end and opposite side edges, wherein said elongated element (A) includes elastic material so as to be elastically stretchable and contractable relative to the outer cover said elongated element (A) being supported between said front and rear sections of said outer cover (1) in a suspended manner to extend longitudinally between said front section and said rear section of the outer cover for spacing the opposite side edges from said outer cover and one or both of the said elastic support members (3, 4) is (are) elastically stretchable and contractible so that when said absorbent article is applied to the wearer, said absorbent member (2) is urged toward a crotch area of the wearer's body.

2. The absorbent article of claim 1, wherein said elastic material comprises a continuous elastic sheet (11) supported between said front and rear sections of the outer cover (1).

3. The absorbent article of claim 2, wherein said elongated element (A) further includes a liquid permeable facing sheet (12), said facing sheet being intermittently secured to said elastic sheet (11) to form a plurality of channels (14) therebetween, said absorbent material being placed in selected ones of said channels.

4. The absorbent article of claim 3, wherein said facing sheet is secured to said elastic sheet (11) through longitudinally spaced, parallel lines of securement (13) for forming the plurality of laterally-extending, parallel channels (14).

5. The absorbent article of claim 4, wherein said absorbent material is placed in the channel (14) in a middle portion of the elongated element (A).

6. The absorbent article of claim 3, wherein said selected ones of the channels (14) have a cross-sectional area greater than that of the remaining channels.

7. The absorbent article of claim 1, wherein one of said front and rear ends of the elongated element (A) is secured to said outer cover (1) by a continuous lateral line of securement (13).

8. The absorbent article of claim 1, wherein one of said front and rear ends of the elongated element (A) is secured to said outer cover (1) by intermittent lateral line of securement (13).

9. The absorbent article of claim 1, wherein said outer cover (1) comprises an elastically stretchable material.

10. The absorbent article of claim 1, wherein said absorbent material comprises superabsorbent material.

11. The absorbent article of claim 1, wherein said elongated element comprises elastic supports (3, 4) connected to both longitudinal ends of said absorbent member (2) and connected to said front section and said rear section of said liquid impermeable outer cover (1), respectively.

12. The absorbent article of daim 11, wherein said front support member (3) is elastically stretchable and contractible.

13. The absorbent article of claim 11, wherein said front and rear support members (3, 4) are both elastically stretchable and contractible.

14. The absorbent article of claim 11, wherein one of said front and rear supports members (3, 4)is liquid impermeable.

15. The absorbent article of claim 11, wherein said elastically stretchable support member includes a plurality of parallel slits (6) respectively extending longitudinally from a longitudinal end thereof to form a plurality of lips separated thereby, each of said lips being secured to the outer cover by a spot of securement (7).

## Patentansprüche

1. Absorbierender Gegenstand für den einmaligen Gebrauch, enthaltend eine für Flüssigkeit undurchlässige äußere Umkleidung (1) mit einem vorderen Bereich und einem hinteren Bereich und ein Absorptionsteil (2), das an der Innenseite der äußeren Umkleidung (1) angebracht ist, **dadurch gekennzeichnet, daß** ein elastischer Träger (3, 4) mit einem Längsende oder daß zwei elastische Träger mit beiden Längsenden des Absorptionsteils (2) unter Bildung eines länglichen Elements (A) mit einem vorderen Ende, einem hinteren Ende und gegenüberliegenden Seitenrändern verbunden ist, wobei das längliche Element (A) elastisches Material einschließt, so daß es gegenüber der äußeren Umkleidung elastisch dehnbar und zusammenziehbar ist, und wobei das längliche Element (A) zwischen dem vorderen und dem hinteren Bereich der äußeren Umkleidung (1) so aufgehängt ist, dass es sich in Längsrichtung zwischen dem vorderen Bereich und dem hinteren Bereich der äußeren Umkleidung erstreckt und die gegenüberliegenden Seitenkanten im Abstand von der äußeren Umkleidung hält, und einer oder beide der elastischen Träger (3, 4) elastisch dehnbar und zusammenziehbar ist (sind), so daß, wenn der absorbierende Artikel von dem Träger angewendet wird, das Absorptionsteil (2) in den Bereich des Schrittes des Körpers des Trägers gezwungen wird.

2. Absorbierender Gegenstand gemäß Anspruch 1, worin das elastische Material eine durchgehende elastische Schicht (11) enthält, die zwischen dem vorderen und dem hinteren Bereich der äußeren Umkleidung (1) angebracht ist.

3. Abosorbierender Gegenstand gemäß Anspruch 2, worin das längliche Element (A) weiter eine für Flüssigkeit durchlässige Überzugsschicht (12) enthält, wobei die Überzugsschicht mit Unterbrechungen an der elastischen Schicht (11) unter Bildung einer Vielzahl von dazwischenliegenden Kanälen (14) befestigt ist, wobei das absorbierende Material in ausgewählten dieser Kanäle eingebracht ist.

4. Absorbierender Gegenstand gemäß Anspruch 3, wobei die Überzugsschicht an der elastischen Schicht (11) durch in Längsrichtung voneinander Abstand haltende, parallele Befestigungslinien (13) zur Bildung der Vielzahl von sich in Querrichtung erstreckenden, parallelen Kanälen (14) befestigt ist.

5. Absorbierender Gegenstand gemäß Anspruch 4, worin das absorbierende Material in den Kanälen (14) im mittleren Bereich des länglichen Elements (A) eingebracht ist.

6. Absorbierender Gegenstand gemäß Anspruch 3, worin die ausgewählten der Kanäle (14) eine größere Querschnittsfläche als die restlichen Kanäle haben.

7. Absorbierender Gegenstand gemäß Anspruch 1, worin eines der vorderen und hinteren Enden des länglichen Elements (A) an der äußeren Umkleidung (1) durch eine durchgehende querlaufende Befestigungslinie (13) befestigt ist.

8. Absorbierender Gegenstand gemäß Anspruch 1, worin eines der vorderen und hinteren Enden des länglichen Elements (A) an der äußeren Umkleidung (1) durch eine unterbrochene querlaufende Befestigungslinie (13) befestigt ist.

9. Absorbierender Gegenstand gemäß Anspruch 1, worin die äußere Umkleidung (1) ein elastisch dehnbares Material enthält.

10. Absorbierender Gegenstand gemäß Anspruch 1, worin das absorbierende Material ein superabsorbierendes Material enthält.

11. Absorbierender Gegenstand gemäß Anspruch 1, worin das längliche Element elastische Träger (3, 4) enthält, die an beide Längsenden des absorbierenden Teils (2) und an den vorderen Bereich bzw. hinteren Bereich der für Flüssigkeit undurchlässigen äußeren Umkleidung (1) gebunden sind.

12. Absorbierender Gegenstand gemäß Anspruch 11, worin das vordere Trägerteil (3) elastisch dehnbar und zusammenziehbar ist.

13. Absorbierender Gegenstand gemäß Anspruch 11, worin sowohl das vordere als auch das hintere Trägerteil (3, 4) elastisch dehnbar und zusammenziehbar sind.

14. Absorbierender Gegenstand gemäß Anspruch 11, worin eines der vorderen und hinteren Trägerteile (3, 4) für Flüssigkeit undurchlässig ist.

15. Absorbierender Gegenstand gemäß Anspruch 11, worin das elastisch dehnbare Trägerteil eine Vielzahl von parallelen Schlitzen (6) enthält, die sich jeweils in Längsrichtung von einem Längsende davon erstrecken, wobei eine Vielzahl von dadurch getrennten Lippen gebildet wird, wobei jede dieser Lippen an der äußeren Umkleidung durch einen Befestigungspunkt (7) befestigt ist.

## Revendications

1. Article absorbant jetable comprenant une couverture externe imperméable au liquide (1) présentant une section avant et une section arrière et un élément absorbant (2) disposé sur le côté interne de ladite couverture externe (1), **caractérisé en ce qu'**un élément de support élastique (3, 4) est raccordé à une extrémité longitudinale ou deux éléments de support élastiques sont raccordés aux deux extrémités longitudinales dudit élément absorbant (2) pour former un élément allongé (A) présentant une extrémité avant, une extrémité arrière et des bords latéraux opposés, dans lequel ledit élément allongé (A) comprend un matériau élastique afin d'être élastiquemenent étirable et contractable par rapport à la couverture externe,
ledit élément allongé (A) étant supporté entre lesdites sections avant et arrière de ladite couverture externe (1) de manière suspendue pour s'étendre longitudinalement entre ladite section avant et ladite section arrière de la couverture externe pour espacer les bords latéraux opposés de ladite couverture externe et un desdits éléments de support élastiques ou les deux (3,4) est(sont) élastiquement étirable(s) et contractable(s) de telle sorte que, lorsque ledit article absorbant est appliqué à l'utilisateur, ledit élément absorbant (2) est poussé vers une zone d'entrejambe du corps de l'utilisateur.

2. Article absorbant selon la revendication 1, dans lequel ledit matériau élastique comprend une feuille élastique continue (11) supportée entre lesdites sections avant et arrière de la couverture externe (1).

3. Article absorbant selon la revendication 2, dans lequel ledit élément allongé (A) comprend en outre une feuille avant perméable au liquide (12), ladite feuille avant étant fixée de manière discontinue à ladite feuille élastique (11) pour former de nombreux canaux (14) entre celles-ci, ledit matériau absorbant étant placé dans des canaux choisis parmi lesdits canaux.

4. Article absorbant selon la revendication 3, dans lequel ladite face avant est fixée à ladite feuille élastique (11) par l'intermédiaire de lignes de fixation parallèles, longitudinalement espacées (13) pour former les nombreux canaux parallèles s'étendant latéralement (14).

5. Article absorbant selon la revendication 4, dans lequel ledit matériau absorbant est placé dans le canal (14) dans une partie milieu de l'élément allongé (A).

6. Article absorbant selon la revendication 3, dans lequel lesdits canaux choisis parmi les canaux (14) présentent une surface transversale supérieure à celle des canaux restants.

7. Article absorbant selon la revendication 1, dans lequel une desdites extrémités avant et arrière de l'élément allongé (A) est fixée à ladite couverture externe (1) par une ligne de fixation latérale continue (13).

8. Article absorbant selon la revendication 1, dans lequel une desdites extrémités avant et arrière de l'élément allongé (A) est fixée à ladite couverture externe (1) par une ligne de fixation latérale discontinue (13).

9. Article absorbant selon la revendication 1, dans lequel ladite couverture externe (1) comprend un matériau élastiquement étirable.

10. Article absorbant selon la revendication 1, dans lequel ledit matériau absorbant comprend un matériau super absorbant.

11. Article absorbant selon la revendication 1, dans lequel ledit élément allongé comprend des supports élastiques (3, 4) raccordés aux deux extrémités longitudinales dudit élément absorbant (2) et raccordés respectivement à ladite section avant et à ladite section arrière de ladite couverture externe imperméable au liquide (1).

12. Article absorbant selon la revendication 11, dans lequel ledit élément de support avant (3) est élastiquement étirable et contractable.

13. Article absorbant selon la revendication 11, dans lequel lesdits éléments de support avant et arrière (3, 4) sont tous deux élastiquement étirables et contractables.

14. Article absorbant selon la revendication 11, dans lequel un desdits éléments de support avant et arrière (3,4) est imperméable au liquide.

15. Article absorbant selon la revendication 11, dans lequel ledit élément de support élastiquement étirable comprend de nombreuses fentes parallèles (6) s'étendant respectivement longitudinalement à partir d'une extrémité longitudinale de celui-ci pour former de nombreuses lèvres séparées par celles-ci, chacune desdites lèvres étant fixée à la couverture externe par un point de fixation 7.
